# EUROPEAN PATENT APPLICATION

(11) **EP 3 327 020 A1**
(43) Date of publication of application: **30.05.2018**
(21) Application number: 16201249.6
(22) Date of filing: 29.11.2016
(51) Int. Cl.: C07D 487/04, A61K 31/519, A61P 19/02

(54) **CITRATE SALTS OF A JANUS KINASE (JAK) INHIBITOR**

(71) Applicant: SANDOZ AG, 4056 Basel (CH)
(72) Inventor: ADAMER, Verena, 6250 Kundl (AT); SCHREINER, Erwin Paul, 6250 Kundl (AT)
(74) Representative: Prüfer & Partner mbB Patentanwälte · Rechtsanwälte

(57) **Abstract**

The present invention relates to citrate salts of baricitinib, in particular to baricitinib mono- and dicitrate, as well as to crystalline and amorphous forms thereof, and to methods for their preparation. Furthermore, the invention relates to a pharmaceutical composition comprising an effective and/or predetermined amount of one or more citrate salts of baricitinib and to the use of said pharmaceutical composition as a medicament, in particular for the treatment of rheumatoid arthritis.

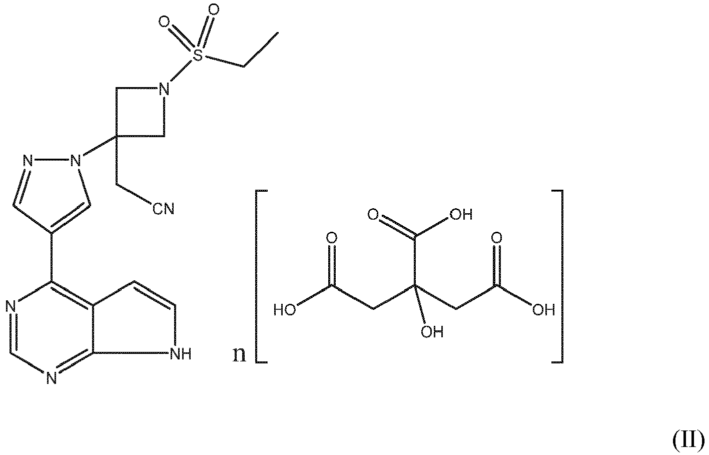

## Description

### FIELD OF THE INVENTION

The present invention relates to citrate salts of baricitinib, in particular to baricitinib mono- and dicitrate, as well as to crystalline and amorphous forms thereof, and to methods for their preparation. Furthermore, the invention relates to a pharmaceutical composition comprising one or more citrate salts of baricitinib, and to the use of said pharmaceutical composition as a medicament, in particular for the treatment of rheumatoid arthritis.

### BACKGROUND OF THE INVENTION

Baricitinib, a Janus Kinase (JAK) inhibitor, is currently in pre-registration for the treatment of rheumatoid arthritis. Chemically designated as {1-(Ethylsulfonyl)-3-[4-(7H-pyrrolo[2,3-d]pyrimidin-4-yl)-1H-pyrazol-1-yl]azetidin-3-yl}acetonitrile, baricitinib may be represented by the chemical structure as depicted in Formula I:

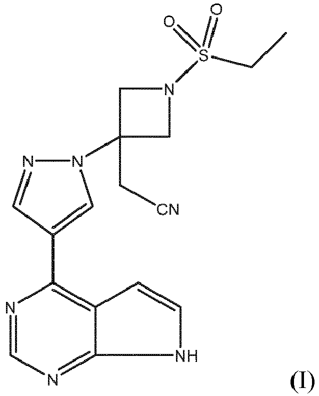

WO 2009/114512 A1 discloses processes for the preparation of baricitinib free base, which is obtained as an off-white solid in unknown form in Examples 70 and 78. In addition, Example 71 of the same application provides a process for the preparation of baricitinib phosphate, which is obtained as a white crystalline solid having a melting point of 187 °C. Finally, a process for the preparation of baricitinib trifluoroacetate is given in Example 1 of WO 2009/114512 A1 which is obtained in unknown state.

CN105601635 discloses crystalline Form A, Form H and Form I of baricitinib phosphate and methods for their preparation. According to the application experimental repetition of Example 71 of WO 2009/114512 resulted in a crystalline form of baricitinib phosphate, which is denominated "Form X" in CN105601635, a PXRD of said "Form X" being displayed in Figure 16 of CN105601635.

In CN105566332 two crystalline forms of baricitinib trifluoroacetate designated Form A and Form B as well as methods for their preparation are disclosed. According to said application repetition of Example 1 of WO 2009/114512 A1 leads to a "semi-crystal" of baricitinib trifluoroacetate, a PXRD of said "semi-crystal" being displayed in Figure 15 of CN CN105566332.

Finally, solid forms of the free base are disclosed in of applications WO 2015/145286 A1, WO 2015/166434 A1, CN105693731 and IP.com article IPCOM000244270D.

It is well known to the person skilled in the art that solubility, dissolution rate and gastrointestinal permeability are fundamental parameters that control rate and extent of drug absorption and its bioavailability. The water solubility of a drug is a crucial property that plays an important role in the absorption of the drug after oral administration. It also governs the possibility of preparing liquid formulations intended for oral or parenteral administration of a drug. While salt formation is a well-known means of enhancing the aqueous solubility of an acidic or basic drug substance, the actual increase in aqueous solubility varies depending on counter-ions. Hence, the identification of a particularly highly soluble salt is not predictable and therefore remains an empirical exercise.

### SUMMARY OF THE INVENTION

The present invention provides citrate salts of baricitinib, showing superior aqueous solubilities and dissolution profiles compared to known baricitinib free base, baricitinib phosphate and baricitinib trifluoro acetate. In particular, the invention refers to baricitinib mono- and dicitrate as well as to crystalline and amorphous forms thereof. For example, the invention relates to a crystalline form of baricitinib monocitrate characterized by having a powder X-ray diffractogram comprising reflections at 2-theta angles of (4.6 ± 0.2)°, (14.7 ± 0.2)° and (18.2 ± 0.2)°, when measured at RT with Cu-Kalpha1,2 radiation having a wavelength of 0.15419 nm. Furthermore, the invention refers to a crystalline form of baricitinib dicitrate characterized by having a powder X-ray diffractogram comprising reflections at 2-theta angles of (5.1 ± 0.2)°, (14.5 ± 0.2)°, (16.5 ± 0.2)°, (23.1 ± 0.2)° and (24.2 ± 0.2)°, when measured at RT with Cu-Kalpha1,2 radiation having a wavelength of 0.15419 nm. The invention further relates to processes for the preparation of the baricitinib citrate salts either in their crystalline or amorphous form. Moreover, the present invention relates to a pharmaceutical composition comprising an effective and/or predetermined amount of one or more baricitinib citrate salts in crystalline and/or amorphous form and one or more pharmaceutically acceptable excipient(s) and to the use of said pharmaceutical composition as a medicament, in particular for the treatment of rheumatoid arthritis.

### Abbreviations

- PXRD: powder X-ray diffractogram
- RH: relative humidity
- w-%: weight percent
- v: volume
- TFA: trifluoroacetate

### Definitions

The term "baricitinib" refers to the compound with the chemical name {1-(Ethylsulfonyl)-3-[4-(7H-pyrrolo[2,3-d]pyrimidin-4-yl)-1H-pyrazol-1-yl]azetidin-3-yl} acetonitrile, which is represented by the chemical structure as depicted in Formula I of the present invention. In the present invention "baricitinib" used in isolation indicates the free base form.

The term "baricitinib citrate" refers to the compound with the chemical name {1-(Ethylsulfonyl)-3-[4-(7H-pyrrolo[2,3-d]pyrimidin-4-yl)-1H-pyrazol-1-yl]azetidin-3-yl}acetonitrile citrate, which is represented by the chemical structure as depicted in Formula (II) of the present invention, wherein n is in the range of from 0.3 to 2.5, preferably from 0.4 to 2.1.

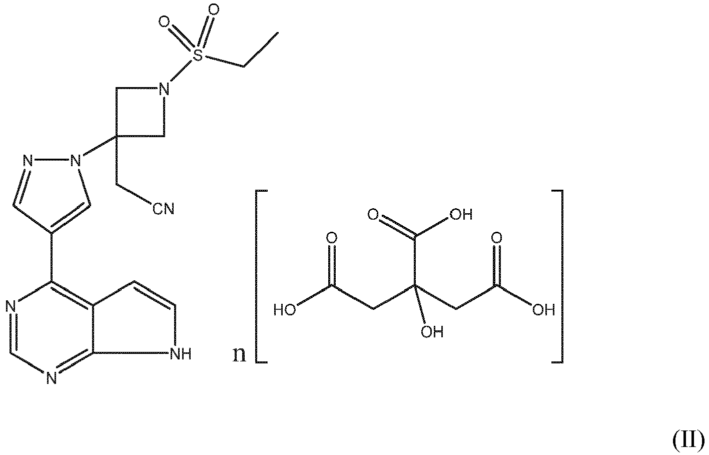

The term "baricitinib monocitrate" refers to the compound with the chemical name {1-(Ethylsulfonyl)-3-[4-(7H-pyrrolo[2,3-d]pyrimidin-4-yl)-1H-pyrazol-1-yl]azetidin-3-yl}acetonitrile monocitrate, which is represented by the chemical structure as depicted in Formula (II) of the present invention, wherein n is in the range of from 0.7 to 1.3, preferably from 0.8 to 1.2, more preferably from 0.9 to 1.1, and most preferably n is 1.0. For example, n may be selected from the group consisting of 0.7, 0.8, 0.9, 1.0, 1.1, 1.2 and 1.3.

The term "baricitinib dicitrate" refers to the compound with the chemical name {1-(Ethylsulfonyl)-3-[4-(7H-pyrrolo[2,3-d]pyrimidin-4-yl)-1H-pyrazol-1-yl]azetidin-3-yl}acetonitrile dicitrate, which is represented by the chemical structure as depicted in Formula (II) of the present invention, wherein n is in the range of from 1.7 to 2.3, preferably from 1.8 to 2.2, more preferably from 1.9 to 2.1, and most preferably n is 2.0. For example, n may be selected from the group consisting of 1.7, 1.8, 1.9, 2.0, 2.1, 2.2 and 2.3.

As used herein the term "room temperature" refers to a temperature in the range of from 20 to 30 °C.

As used herein, the term "measured at a temperature in the range of from 20 to 30 °C" refers to a measurement under standard conditions. Typically, standard conditions mean a temperature in the range of from 20 to 30 °C, i.e. at room temperature, such as 22 °C. Typically, standard conditions can additionally mean a measurement under 35-65% relative humidity, such as 50% relative humidity.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 103 to 1020 atoms, whereas short-range order is over a few atoms only (see "Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to PXRD means that variabilities in peak positions and relative intensities of the peaks are to be taken into account. For example, a typical precision of the 2-Theta values is in the range of ± 0.2° 2-Theta, preferably in the range of ± 0.1 2-Theta. Thus, a diffraction peak that usually appears at 4.6° 2-Theta for example can appear between 4.4° and 4.8° 2-Theta, preferably between 4.5° and 4.7° 2-Theta on most X-ray diffractometers under standard conditions. Furthermore, one skilled in the art will appreciate that relative peak intensities will show inter-apparatus variability as well as variability due to degree of crystallinity, preferred orientation, sample preparation and other factors known to those skilled in the art and should be taken as qualitative measure only.

As used herein, the term "substantially pure" with reference to a particular polymorphic form means that the polymorphic form includes less than 10%, preferably less than 5%, more preferably less than 3%, most preferably less than 1% by weight of any other physical forms of the compound. The presence of more than one polymorph in a sample may be determined by techniques such as x-ray powder diffraction (XRPD) or solid state nuclear magnetic resonance spectroscopy. For example, the presence of extra peaks in the comparison of an experimentally measured XRPD pattern with a simulated XRPD pattern may indicate more than one polymorph in the sample. The simulated XRPD may be calculated from single crystal x-ray data. see Smith, D.K., "A FORTRAN Program for Calculating X-Ray Powder Diffraction Patterns," Lawrence Radiation Laboratory, Livermore, California, UCRL-7196 (April 1963) or TOPAS program (Total Pattern Analysis Solution, available through Brucker AXS Inc.).

The term "solid form" as used herein refers to any crystalline and amorphous phase of a substance.

A "predetermined amount" as used herein with regard to baricitinib citrate of the present invention refers to the initial amount of baricitinib citrate used for the preparation of a pharmaceutical composition.

The term "effective amount" as used herein with regard to baricitinib citrate of the present invention encompasses an amount of baricitinib citrate, which causes the desired therapeutic and/or prophylactic effect.

The term "about" as used herein means within 5%, more typically within 1% and most typically within 0.5% of the indicated value or range.

As used herein "amorphous" refers to a solid form of a molecule, atom, and/or ions that is not crystalline.

Baricitinib citrate may be referred to herein as being characterized by graphical data "as shown in" a Figure. Such data can include, for example, powder X-ray difractograms (XRPD), differential scanning calorimetry (DSC) thermograms and thermogravimetric analysis (TGA) . The person skilled in the art understands that factors such as variations in instrument type, response and variations in sample directionality, sample concentration and sample purity may lead to small variations for such data when presented in graphical form, for example variations relating to the exact peak positions and intensities. However, a comparison of the graphical data in the Figures herein with the graphical data generated for an unknown solid form and the confirmation that two sets of graphical data relate to the same crystal form is well within the knowledge of a person skilled in the art.

As used herein, the term "mother liquor" refers to the solution remaining after crystallization of a solid.

### BRIEF DESCRIPTION OF THE DRAWINGS

Figure 1: illustrates a representative PXRD of the crystalline form of baricitinib monocitrate of the present invention. The x-axis shows the scattering angle in °2-theta, the y-axis shows the intensity of the scattered X-ray beam in counts of detected photons.
Figure 2: illustrates a representative PXRD of the amorphous form of baricitinib monocitrate of the present invention. The x-axis shows the scattering angle in °2-theta, the y-axis shows the intensity of the scattered X-ray beam in counts of detected photons.
Figure 3: illustrates a representative PXRD of the crystalline form of baricitinib dicitrate of the present invention. The x-axis shows the scattering angle in °2-theta, the y-axis shows the intensity of the scattered X-ray beam in counts of detected photons.
Figure 4: illustrates a representative PXRD of the amorphous form of baricitinib dicitrate of the present invention. The x-axis shows the scattering angle in °2-theta, the y-axis shows the intensity of the scattered X-ray beam in counts of detected photons.

### DETAILED DESCRIPTION OF THE INVENTION

The present invention provides citrate salts of baricitinib. The citrate salts of baricitinib of the present invention show superior aqueous solubilities and/or dissolution profiles compared to known baricitinib free base, baricitinib phosphate and baricitinib trifluoroacetate. High solubility is the key factor for timely and complete in-vivo dissolution of the active pharmaceutical ingredient contained in an oral solid dosage form, e.g., an immediate-release tablet or a hard gelatin capsule. In addition, aqueous solubility of a drug substance is the most crucial factor in designing liquid drug products because the drug substance is applied in solution. Due to their extraordinary high aqueous solubilities the baricitinib citrate salts of the present invention are very well suited for the preparation of aqueous solutions intended for parenteral or oral administration.

Different aspects of the invention are described below in further detail by embodiments, without being limited thereto. Each aspect of the invention may be described by one embodiment or by combining two or more of the embodiments.

In a first aspect, the invention relates to baricitinib citrate.

In one embodiment the invention relates to baricitinib monocitrate, characterized by the chemical structure according to Formula (II),
wherein n is in the range of from 0.7 to 1.3, preferably from 0.8 to 1.2, more preferably from 0.9 to 1.1, and most preferably n is 1.0. In one embodiment n is selected from the group consisting of 0.7, 0.8, 0.9, 1.0, 1.1, 1.2 and 1.3.

In another embodiment the invention relates to baricitinib monocitrate as defined above in amorphous or crystalline form.

The crystalline and amorphous forms of baricitinib monocitrate of the present invention may be characterized by PXRD. In particular, they may be characterized by any one of the following embodiments or by combining two or more of the following embodiments.

Hence, in a further embodiment the present invention relates to a crystalline form of baricitinib monocitrate characterized by having a PXRD comprising reflections at 2-Theta angles of:
(4.6 ± 0.2)°, (14.7 ± 0.2)° and (18.2 ± 0.2)°; or
(4.6 ± 0.2)°, (14.7 ± 0.2)°, (16.0 ± 0.2)° and (18.2 ± 0.2)°; or
(4.6 ± 0.2)°, (14.7 ± 0.2)°, (16.0 ± 0.2)°, (18.2 ± 0.2)° and (26.4 ± 0.2)°; or
(4.6 ± 0.2)°, (4.9 ± 0.2)°, (14.7 ± 0.2)°, (16.0 ± 0.2)°, (18.2 ± 0.2)° and (26.4 ± 0.2)°; or
(4.6 ± 0.2)°, (4.9 ± 0.2)°, (14.7 ± 0.2)°, (16.0 ± 0.2)°, (18.2 ± 0.2)°, (18.6 ± 0.2)° and (26.4 ± 0.2)°; or
(4.6 ± 0.2)°, (4.9 ± 0.2)°, (14.7 ± 0.2)°, (16.0 ± 0.2)°, (18.2 ± 0.2)°, (18.6 ± 0.2)°, (18.9 ± 0.2)° and (26.4 ± 0.2)°; or
(4.6 ± 0.2)°, (4.9 ± 0.2)°, (12.9 ± 0.2)°, (14.7 ± 0.2)°, (16.0 ± 0.2)°, (18.2 ± 0.2)°, (18.6 ± 0.2)°, (18.9 ± 0.2)° and (26.4 ± 0.2)°; or
(4.6 ± 0.2)°, (4.9 ± 0.2)°, (9.9 ± 0.2)°, (12.9 ± 0.2)°, (14.7 ± 0.2)°, (16.0 ± 0.2)°, (18.2 ± 0.2)°, (18.6 ± 0.2)°, (18.9 ± 0.2)° and (26.4 ± 0.2)°;
when measured at RT with Cu-Kalpha1,2 radiation having a wavelength of 0.15419 nm.

Preferably said crystalline form of baricitinib monocitrate is substantially pure.

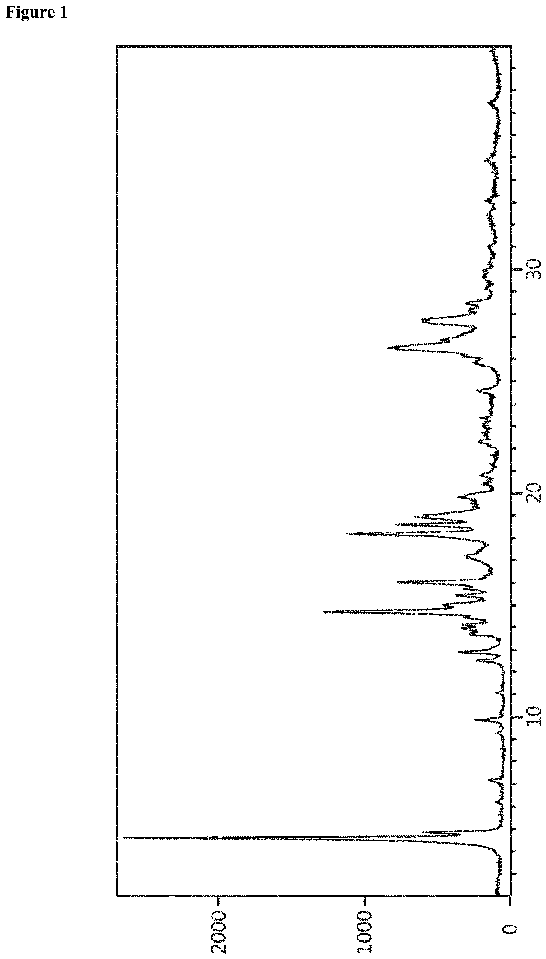

In another embodiment, the present invention relates to a crystalline form of baricitinib monocitrate characterized by having a PXRD essentially the same as shown in Figure 1 of the present invention, when measured at RT with Cu-Kalpha1,2 radiation having a wavelength of 0.15419 nm.

Another embodiment of the present invention concerns an amorphous form of baricitinib monocitrate, characterized by having a PXRD comprising no reflection in the range of from 2 to 40° 2-Theta, when measured at RT with Cu-Kalpha1,2 radiation having a wavelength of 0.15419 nm.

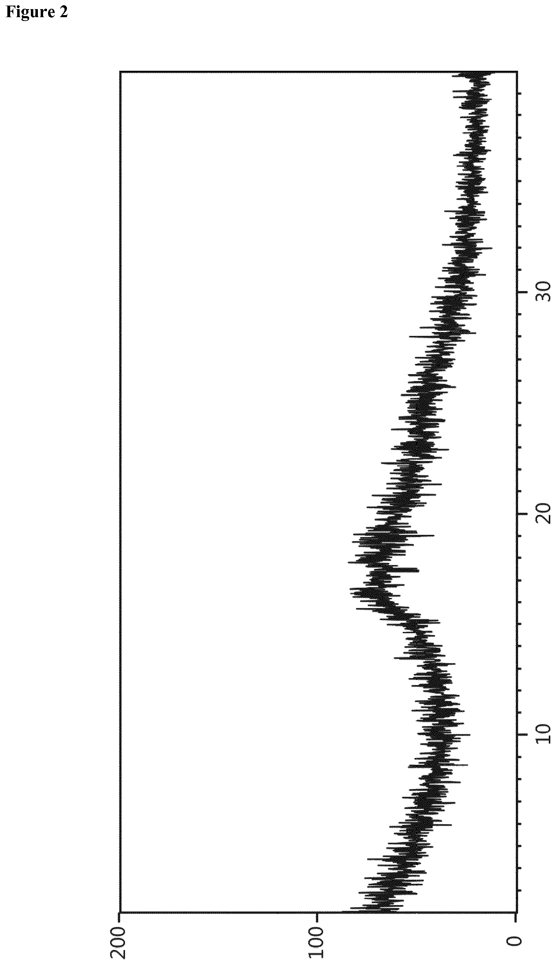

In still another embodiment, the invention relates to an amorphous form of baricitinib monocitrate characterized by having a PXRD essentially the same as displayed in Figure 2 of the present invention, when measured at room temperature with CuKalpha11,2 radiation having a wavelength of 0.15419 nm.

In a further embodiment the invention relates to baricitinib dicitrate, characterized by the chemical structure according to Formula (II),
wherein n is in the range of from 1.7 to 2.3, preferably from 1.8 to 2.2, more preferably from 1.9 to 2.1, and most preferably n is 2.0. In one embodiment n is selected from the group consisting of 1.7, 1.8, 1.9, 2.0, 2.1, 2.2 and 2.3.

In another embodiment the invention relates to baricitinib dicitrate as defined above in amorphous or crystalline form.

The crystalline and amorphous forms of baricitinib dicitrate of the present invention may be characterized by PXRD. In particular, they may be characterized by any one of the following embodiments or by combining two or more of the following embodiments.

In one embodiment the present invention relates to a crystalline form of baricitinb dicitrate characterized by having a PXRD comprising reflections at 2-Theta angles of:
(5.1 ± 0.2)°, (14.5 ± 0.2)°, (16.5 ± 0.2)°, (23.1 ± 0.2)° and (24.2 ± 0.2)°; or
(5.1 ± 0.2)°, (14.5 ± 0.2)°, (16.5 ± 0.2)°, (18.8 ± 0.2)°, (23.1 ± 0.2)° and (24.2 ± 0.2)°; or
(5.1 ± 0.2)°, (6.2 ± 0.2)°, (14.5 ± 0.2)°, (16.5 ± 0.2)°, (18.8 ± 0.2)°, (23.1 ± 0.2)° and (24.2 ± 0.2)°; or
(5.1 ± 0.2)°, (6.2 ± 0.2)°, (6.8 ± 0.2)°, (14.5 ± 0.2)°, (16.5 ± 0.2)°, (18.8 ± 0.2)°, (23.1 ± 0.2)° and (24.2 ± 0.2)°; or
(5.1 ± 0.2)°, (6.2 ± 0.2)°, (6.8 ± 0.2)°, (10.3 ± 0.2)°, (14.5 ± 0.2)°, (16.5 ± 0.2)°, (18.8 ± 0.2)°, (23.1 ± 0.2)° and (24.2 ± 0.2)°; or
(5.1 ± 0.2)°, (6.2 ± 0.2)°, (6.8 ± 0.2)°, (10.3 ± 0.2)°, (12.6 ± 0.2)°, (14.5 ± 0.2)°, (16.5 ± 0.2)°, (18.8 ± 0.2)°, (23.1 ± 0.2)° and (24.2 ± 0.2)°;
when measured at RT with Cu-Kalpha1,2 radiation having a wavelength of 0.15419 nm. Preferably said crystalline form of baricitinib dicitrate is substantially pure.

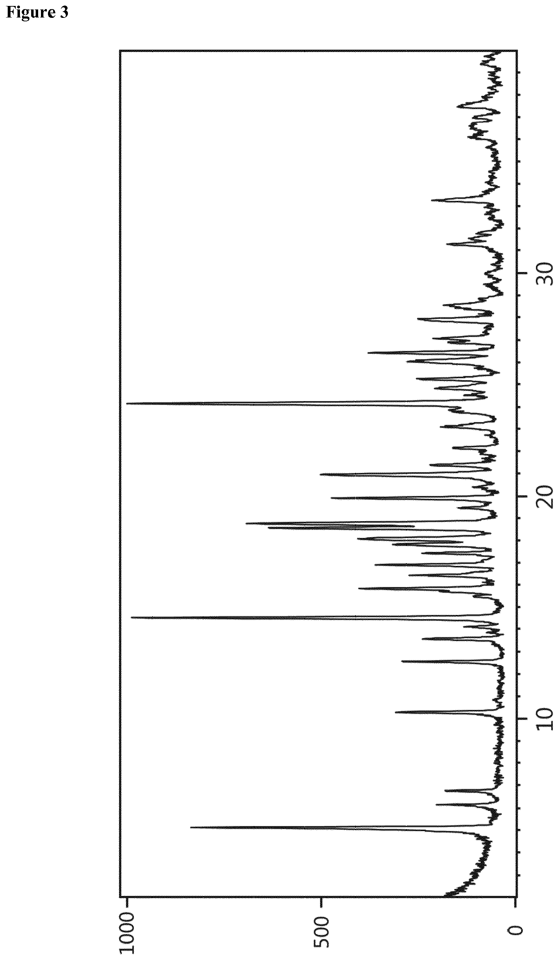

In still another embodiment, the present invention relates to a crystalline form of baricitinib dicitrate characterized by having a PXRD essentially the same as shown in Figure 3 of the present invention, when measured at RT with Cu-Kalpha1,2 radiation having a wavelength of 0.15419 nm.

Another embodiment of the present invention concerns an amorphous form of baricitinib dicitrate, characterized by having a PXRD comprising no reflection in the range of from 2 to 40° 2-Theta, when measured at RT with CuKalpha1,2 radiation having a wavelength of 0.15419 nm.

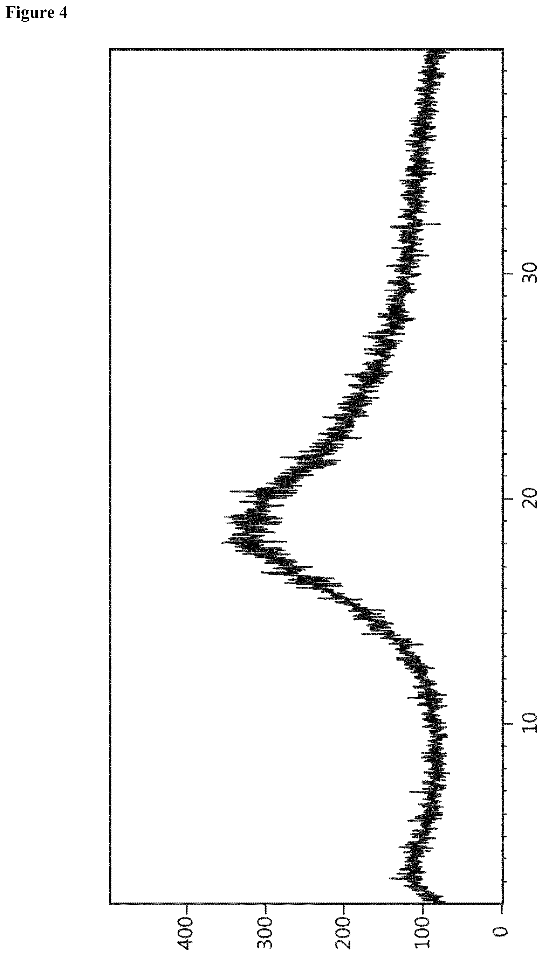

In still another embodiment, the invention relates to an amorphous form of baricitinib dicitrate characterized by having a PXRD essentially the same as displayed in Figure 4 of the present invention, when measured at room temperature with CuKalpha1,2 radiation having a wavelength of 0.15419 nm.

The citrate salts of baricitinib of the present invention show improved aqueous solubilities and dissolution rates compared to baricitinib free base, baricitinib phosphate and baricitinib trifluoroacetate. Comparative Example 1 of the present invention for example provides solubility data of the aforementioned salts in 0.1 N aqueous hydrochloric acid, a medium simulating the pH environment of the stomach. Surprisingly, the citrate salts of baricitinib of the present invention, show a solubility, which is more than 2-fold higher than the solubility of baricitinib free base. In contrast, the phosphate salt as well as the trifluoroacetate salt of baricitinib show only slightly improved solubility values compared to the free base form in the same media.

In a further aspect, the invention relates to a process for the preparation of crystalline baricitinib monocitrate as defined above comprising the steps of:
(i) suspending amorphous baricitinib monocitrate in one or more aprotic solvent(s); or
(ii) reacting one mol equivalent baricitinib free base with 0.7 to 1.3 mol equivalent citric acid in one or more aprotic solvent(s); and
(iii)slurrying the mixture obtained in step (i) or (ii); and
(iv)optionally separating at least a part of the crystals obtained in step (iii) from their mother liquor; and
(v) optionally, drying the crystals obtained in step (iii) or (iv);

Amorphous baricitinib monocitrate, which is applied as starting material in step (i) of the above described process may be prepared by the lyophilization process described hereinafter for the preparation of amorphous baricitinib monocitrate. Baricitinib free base, which is used as starting material in step (ii) of the above described process may be prepared according to the procedures disclosed in Example 70 and 78 of WO 2009/114512 A1 respectively. Citric acid is commercially available.

In one embodiment the one or more aprotic solvent(s) may be selected from the group consisting of nitriles, esters, ketones and hydrocarbons. Concrete examples for aprotic solvents, which may be used are for example but not limited to acetonitrile, methyl acetate, ethyl acetate, methyl ethyl ketone, cyclohexanone and cyclohexane, whereat acetonitrile is most preferred. The final baricitinib citrate concentration of the mixture obtained in step (i) or (ii) is preferably in the range of from 50 to 500 g/L, more preferably from 100 to 300 g/L such as 100 to 200 g/L.

The mixture obtained in step (i) or (ii) is slurried in step (iii), wherein slurrying in the context of the present invention relates to any motion of the mixture comprising baricitinib monocitrate, which is caused by stirring, shaking and/or ultrasonic irradiation. Slurrying is performed until at least a part, preferably all of the baricitinib monocitrate has transformed to the crystalline form of the present invention. The completeness of the transformation can be monitored by taking samples and investigating the samples by PXRD. Usually, slurrying is performed for a period in the range of from about 6 to 48 hours, preferably from about 12 to 36 hours.

The obtained baricitinib monocitrate crystals or at least a part thereof may optionally be separated from their mother liquor by any conventional method such as filtration or centrifugation, most preferably by filtration. Optionally, the isolated crystals may be washed with a solvent, preferably with one or more aprotic solvent(s).

Finally, the baricitinib monocitrate crystals may optionally be dried at a temperature of about 80 °C or less, preferably of about 60 °C or less, more preferably of about 40 °C or less and most preferably the crystals are dried at RT. Drying may be performed for a period in the range of from about 1 to 72 hours, preferably from about 2 to 48 hours, more preferably from about 4 to 24 hours and most preferably from about 6 to 18 hours. Drying may be performed at ambient pressure and/or under vacuum preferably at about 100 mbar or less, more preferably at about 50 mbar or less and most preferably at about 30 mbar or less, for example at about 20 mbar or less.

In another aspect, the invention relates to a process for the preparation of amorphous baricitinib monocitrate as defined above comprising:
(i) reacting one mol equivalent baricitinib free base with 0.7 to 1.3 mol equivalent citric acid in the presence of one or more aqueous water miscible organic solvent(s) or 1,4-dioxane; and
(ii) lyophilizing the solution provided in step (i);

The one or more water miscible organic solvent(s) may be selected from the group consisting of ethanol, acetonitrile and 1,4-dioxane. Non-limiting examples and conditions of the lyophilization process are disclosed in Example 2 of the present invention.

In a further aspect, the invention relates to a process for the preparation of crystalline baricitinib dicitrate as defined above comprising the steps of:
(i) suspending amorphous baricitinib dicitrate in one or more aprotic solvent(s); and
(ii) slurrying the mixture obtained in step (i); and
(iii)optionally separating at least a part of the crystals obtained in step (ii) from their mother liquor; and
(iv)optionally, drying the crystals obtained in step (ii) or (iii);

Amorphous baricitinib dicitrate, which is applied as starting material in step (i) of the above described process may be prepared either by the lyophilization process described hereinafter for the preparation of amorphous baricitinib dicitrate or by reacting one mol equivalent of baricitinib free base with 2.0 to 6.0 mol equivalent, preferably 3.0 to 5.0 mol equivalent citric acid in the presence of one or more aprotic solvent(s), followed by evaporation of the solvent. Baricitinib free base, which is used as starting material in step (i) of the above described process may be prepared according to the procedures disclosed in Example 70 and 78 of WO 2009/114512 A1 respectively. Citric acid is commercially available.

In one embodiment the one or more aprotic solvent(s) may be selected from the group consisting of nitriles, esters, ketones and hydrocarbons. Concrete examples for aprotic solvents, which may be used are for example but not limited to acetonitrile, methyl acetate, ethyl acetate, methyl ethyl ketone, cyclohexanone and cyclohexane, whereat acetonitrile is most preferred. The final baricitinib citrate concentration of the mixture obtained in step (i) or (ii) is preferably in the range of from 100 to 700 g/L, more preferably of from 100 to 500g/L such as 100 to 350 g/L.

The mixture obtained in step (i) is slurried in step (ii), wherein slurrying in the context of the present invention relates to any motion of the mixture comprising baricitinib dicitrate, which is caused by stirring, shaking and/or ultrasonic irradiation. Slurrying is performed until at least a part, preferably all of the baricitinib dicitrate has transformed to the crystalline form of the present invention. The completeness of the transformation can be monitored by taking samples and investigating the samples by PXRD. Usually, slurrying is performed for a period in the range of from about 6 to 48 hours, preferably from about 12 to 36 hours.

The obtained baricitinib dicitrate crystals or at least a part thereof may optionally be separated from their mother liquor by any conventional method such as filtration or centrifugation, most preferably by filtration. Optionally, the isolated crystals may be washed with a solvent, preferably with one or more aprotic solvent(s).

Finally, the baricitinib dicitrate crystals may optionally be dried at a temperature of about 80 °C or less, preferably of about 60 °C or less, more preferably of about 40 °C or less and most preferably the crystals are dried at RT. Drying may be performed for a period in the range of from about 1 to 72 hours, preferably from about 2 to 48 hours, more preferably from about 4 to 24 hours and most preferably from about 6 to 18 hours. Drying may be performed at ambient pressure and/or under vacuum preferably at about 100 mbar or less, more preferably at about 50 mbar or less and most preferably at about 30 mbar or less, for example at about 20 mbar or less.

In another aspect, the invention relates to a process for the preparation of amorphous baricitinib dicitrate as defined above comprising:
(i) reacting one mol equivalent baricitinib free base with 1.8 to 6.0 mol equivalent citric acid in the presence of one or more aqueous water-miscible organic solvent(s) or 1,4-dioxane; and
(ii) lyophilizing the solution provided in step (i);

The one or more water miscible organic solvent(s) may be selected from the group consisting of ethanol, acetonitrile and 1,4-dioxane. Non-limiting examples and conditions of the lyophilization process are disclosed in Example 4 of the present invention.

In a further aspect the invention relates to a pharmaceutical composition comprising baricitinib citrate as defined above and one or more pharmaceutically acceptable excipient(s). In particular the pharmaceutical composition can be a pharmaceutical dosage form, such as an oral pharmaceutical dosage form, preferably a tablet.

The invention also relates to a pharmaceutical composition, in particular a pharmaceutical dosage form such as a capsule or a tablet, comprising
(a) baricitinib citrate, and
(b) one or more excipient(s) selected from
   10 to 95 w-% of a filler,
   1 to 25 w-% of a disintegrant,
   a lubricant, and a glidant,
   wherein w-% ages are based upon the total weight of the composition.

The pharmaceutical composition comprises baricitinib, such as a baricitinib citrate of the present invention, preferably in an amount of from 0.5 to 25 w-%, preferably between 1 and 20 w-%, more preferably between 2 and 10 w-%, based on the total weight of the composition.

The pharmaceutical composition further comprises at least one excipient. Suitable excipients include, but are not limited to, fillers, lubricants, disintegrants, surfactants, glidants, antisticking agents and mixtures thereof.

Generally, fillers are used to top up the volume for an appropriate oral deliverable dose, when low concentrations of the active pharmaceutical ingredients (about 25 w-% or lower) are present. In powder formulations fillers can enhance the powder flow, facilitating for example, a uniform filling of capsules. Examples of fillers are calcium phosphate, calcium carbonate, magnesium carbonate, dextrose, saccharose and lactose, to name but a few.

Preferred amounts of fillers are 10 to 95 w-%, preferably of 20 to 95 w-%, more preferably 40 to 95 w-%, in particular 60 to 90 w-%, based on the total weight of the composition.

The pharmaceutical composition of the present invention may further comprise one or more disintegrants. Generally, disintegrants are compounds which can promote the break up of a solid composition into smaller pieces upon contact with water. Examples of disintegrants are sodium carboxymethyl starch, cross-linked polyvinylpyrrolidone (crospovidone) and sodium carboxymethyl glycolate, to name but a few. Disintegrants may be used in an amount of 1 to 20 w-%, preferably of 1 to 10 w-%, based on the total weight of the composition.

The pharmaceutical composition of the present invention may further comprise a surfactant, for example sodium lauryl sulfate, such as in an amount of 0.1 to 1.5 w-%, based on the total weight of the composition.

Additionally, the pharmaceutical composition, preferably the pharmaceutical dosage form, may comprise one or more additional excipients as for example: a lubricant and/or a glidant.

Lubricants can reduce friction during processing steps. Examples of a lubricant are stearate, talcum powder or a fatty acid. Lubricant may be present in an amount of from 0.1 to 4 w-%, such as from 1.0 to 2.5 w-%of the total weight of the composition.

An example of a glidant is colloidal silicone dioxide, which may be present in an amount of from 0.1 to 3 w-% of the total weight of the composition.

Due to the nature of pharmaceutical excipients, it cannot be excluded that a certain compound meets the functional requirements of more than one of the above mentioned excipient classes. However in the context of the present application, the same pharmaceutical compound can only be subsumed as one of the functional excipient classes presented above. For example, if sodium carboxymethyl starch is described as a disintegrant, it cannot additionally classify as a filler.

The process for producing the pharmaceutical composition of the present invention may comprise the steps of
providing baricitinib, such as baricitinib citrate of the present invention, and at least one excipient, and blending baricitinib and at least one excipient to obtain a composition.

The blend may be agglomerated into granules, and optionally compressed into tablets or filled into capsules.

In still another aspect the invention relates to the pharmaceutical composition as defined above for use as a medicament.

In a further aspect the invention relates to the pharmaceutical composition as defined above for use in the treatment of one or more condition(s) selected from the group consisting of atopic dermatitis, inflammatory disease, rheumatoid arthritis, systemic lupus erythematosus, diabetic nephropathy and psoriasis. In a particular preferred embodiment, the present invention relates to the pharmaceutical composition as defined above for use in the treatment of rheumatoid arthritis.

The following non-limiting examples are illustrative of the disclosure and are not to be interpreted to be in any way limiting for the scope of the invention.

### EXAMPLES

### Powder X-ray diffraction (PXRD)

PXRD was performed with a PANalytical X'Pert PRO diffractometer equipped with a theta/theta coupled goniometer in transmission geometry, Cu-Kalpha1,2 radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions. A typical precision of the 2-Theta values is in the range of ± 0.2° 2-Theta, preferably in the range of ± 0.1 2-Theta. Thus, the reflection of the crystalline form of baricitinib monocitrate that appears for example at 4.6° 2-Theta can appear between 4.4° and 4.8° 2-Theta, preferably between 4.5° and 4.7° 2-Theta on most X-ray diffractometers under standard conditions. The reflection of the crystalline form of baricitinib dicitrate that appears for example at 5.1° 2-Theta can appear between 4.9° and 5.3° 2-Theta, preferably between 5.0° and 5.2° 2-Theta on most X-ray diffractometers under standard conditions.

### Example 1: Preparation of crystalline baricitinib monocitrate

### Example 1.1

Baricitinib free base (401 mg, 1.1 mmol, e.g. prepared according to the teaching of WO 2009/114512 A1, Examples 70 or 78) and citric acid (229 mg, 1.2 mmol) were suspended in acetonitrile (5 mL) and slurried at room temperature for 18 hours. The solid was collected by filtration and dried under vacuum at room temperature for 24 hours. Yield: 373 mg (61% of theory)

A representative powder X-ray diffractogram of the crystalline form of baricitinib monocitrate of the present invention obtained by the process according to Example 1 is displayed in Figure 1 and the corresponding reflection list is provided in Table 1 below.

**Table 1: Reflection list and corresponding relative intensities of the crystalline form of baricitinib monocitrate of the present invention between 2.0 and 30.0° 2-Theta**

| Angle | Relative Intensity | Angle | Relative Intensity |
|---|---|---|---|
| [± 0.2 °2-Theta] | [%] | [± 0.2 °2-Theta] | [%] |
| 4.6 | 100 | 16.0 | 29 |
| 4.9 | 19 | 17.2 | 9 |
| 6.2 | 1 | 18.2 | 42 |
| 7.2 | 3 | 18.6 | 28 |
| 9.3 | 1 | 18.9 | 22 |
| 9.9 | 9 | 19.8 | 11 |
| 11.1 | 2 | 20.4 | 4 |
| 12.5 | 7 | 20.8 | 5 |
| 12.9 | 11 | 22.3 | 6 |
| 13.7 | 9 | 24.6 | 6 |
| 14.0 | 11 | 25.8 | 6 |
| 14.1 | 10 | 26.4 | 28 |
| 14.7 | 49 | 27.6 | 18 |
| 15.0 | 15 | 28.5 | 9 |
| 15.5 | 11 | 29.2 | 4 |
| 15.7 | 10 | 29.7 | 4 |

### Example 1.2

Amorphous baricitinib monocitrate (101 mg, prepared according to Example 2.1 herein) was slurried in acetonitrile (1 mL) at RT using a magnetic stirrer. After 3 days the solid was collected by filtration and dried under vacuum (35 mbar) at room temperature to yield the crystalline form of baricitinib monocitrate of the present invention.

### Example 2: Preparation of amorphous baricitinib monocitrate

### Example 2.1

Baricitinib free base (223 mg, 0.6 mmol, e.g. prepared according to the teaching of WO 2009/114512 A1, Examples 70 or 78) and citric acid (118 mg, 0.6 mmol) were dissolved in aqueous acetonitrile (30 mL, 75% v/v) and subjected to the lyophilization conditions as set out below.

### Example 2.2

Baricitinib free base (214 mg, 0.6 mmol, e.g. prepared according to the teaching of WO 2009/114512 A1, Examples 70 or 78) and citric acid (114 mg, 0.6 mmol) were dissolved in 1,4-dioxane (30 mL) and subjected to the lyophilization conditions as set out below.

### Example 2.3

Baricitinib free base (215 mg, 0.6 mmol, e.g. prepared according to the teaching of WO 2009/114512 A1, Examples 70 or 78) and citric acid (114 mg, 0.6 mmol) were dissolved in a mixture of aqueous ethanol (50 mL, 80% v/v) and 1,4-dioxane (10 mL) and subjected to the lyophilization conditions as set out below.

### Lyophilization

Lyophilization was performed with a Christ alpha 2-4 LSC plus lyophilizer. The following lyophilization program according to Table 3 was applied.

| | **temperature [°C]** | **pressure [mbar]** | **time [hh:mm]** |
|---|---|---|---|
| **freezing** | -30 | ambient | 01:30 |
| **main drying** | -30 | ambient - 0.120 (ramp) | 00:05 |
| | -30 | 0.120 | 10:00 |

**Table 2: Lyophilization program**

| | | | |
|---|---|---|---|
| | -30 - -5 (ramp) | 0.120 | 02:00 |
| | -5 | 0.120 | 10:00 |
| **post drying** | 40 | 0.120 - 0.0010 (ramp) | 02:00 |
| | 40 | 0.0010 | 15:00 |

A representative powder X-ray diffractogram of amorphous baricitinib monocitrate prepared according to the above described process is displayed in Figure 2.

### Example 3: Preparation of crystalline baricitinib dicitrate

### Example 3.1

Baricitinib free base (533 mg, 1.4 mmol, e.g. prepared according to the teaching of WO 2009/114512 A1, Examples 70 or 78) and citric acid (1109 mg, 5.8 mmol) were dissolved in acetonitrile (10 mL) upon heating to reflux. The clear solution was cooled to 0 °C in 2 hours whereupon a turbid solution was obtained. Subsequently, the solvent was removed in vacuo (rotary evaporator: 30 mbar, 40 °C bath temperature). The obtained residue was suspended in acetonitrile (5 mL) and treated in an ultrasonic bath for 15 min. The suspension was then slurried at room temperature for 18 hours. The solid was collected by filtration and dried under vacuum (35 mbar) at 40 °C for 24 hours. Yield: 722 mg (67% of theory)

A representative powder X-ray diffractogram of the crystalline form of baricitinib dicitrate of the present invention prepared according to Example 3.1 is displayed in Figure 3 and the corresponding reflection list is provided in Table 3 below.

**Table 3: Reflection list and corresponding relative intensities of the crystalline form of baricitinib dicitrate of the present invention between 2.0 and 30.0° 2-Theta**

| Angle | Relative Intensity | Angle | Relative Intensity |
|---|---|---|---|
| [± 0.2 °2-Theta] | [%] | [± 0.2 °2-Theta] | [%] |
| 5.1 | 81 | 19.9 | 45 |
| 6.2 | 16 | 20.4 | 6 |
| 6.8 | 14 | 21.0 | 48 |
| 10.3 | 27 | 21.4 | 19 |
| 12.6 | 26 | 22.1 | 12 |
| 13.6 | 21 | 23.1 | 16 |
| 14.1 | 9 | 23.8 | 12 |
| 14.5 | 100 | 24.2 | 98 |
| 15.5 | 7 | 24.8 | 17 |
| 15.8 | 38 | 25.3 | 21 |
| 16.5 | 21 | 26.1 | 22 |
| 16.9 | 34 | 26.4 | 36 |
| 17.4 | 22 | 26.9 | 14 |
| 17.8 | 28 | 27.1 | 17 |
| 18.1 | 38 | 27.9 | 22 |
| 18.6 | 63 | 28.6 | 13 |
| 18.8 | 68 | 29.5 | 3 |
| 19.5 | 12 | 30.0 | 4 |

### Example 3.2

Baricitinib free base (156 mg, 0.4 mmol, e.g. prepared according to the teaching of WO 2009/114512 A1, Examples 70 or 78) was dissolved in acetonitrile (5 mL) upon heating to reflux. To the hot solution a solution of citric acid (670 microL, 1.4 mmol) in ethanol (2.08 mol/L) were added and the solution was subsequently cooled to room temperature at a rate of - 10 K/min. The solvent of was removed in vacuo (rotary evaporator: 30 mbar, 40 °C bath temperature) and the obtained residue was suspended in acetonitrile (0.5 mL). The suspension was treated in an ultrasonic bath for 5 min and subsequently stirred at room temperature for 18 hours. The solid was collected by filtration and dried under vacuum (35 mbar) at room temperature for 24 hours.

### Yield: 192 mg (61 % of theory)

### Example 3.3

Amorphous baricitinib dicitrate (159 mg, prepared according to Example 4.1 herein) was slurried in acetonitrile (1.5 mL) at RT using a magnetic stirrer. After 3 days the solid was collected by filtration and dried under vacuum (35 mbar) at room temperature to obtain the crystalline form of baricitinib dicitrate of the present invention.

### Example 4: Preparation of amorphous baricitinib dicitrate

### Example 4.1

Baricitinib free base (210 mg, 0.6 mmol, e.g. prepared according to the teaching of WO 2009/114512 A1, Examples 70 or 78) and citric acid (218 mg, 1.1 mmol) were dissolved in in a mixture of aqueous ethanol (60 mL, 20% v/v) and 1,4-dioxane (10 mL) and subjected to the lyophilization conditions as set out below.

### Example 4.2

Baricitinib free base (216 mg, 0.6 mmol, e.g. prepared according to the teaching of WO 2009/114512 A1, Examples 70 or 78) and citric acid (225 mg, 1.2 mmol) were dissolved in 1,4-dioxane (50 mL) and subjected to the lyophilization conditions as set out below.

### Example 4.3

Baricitinib free base (230 mg, 0.6 mmol, e.g. prepared according to the teaching of WO 2009/114512 A1, Examples 70 or 78) and citric acid (238 mg, 1.2 mmol) were dissolved in a aqueous acetonitrile (30 mL, 75% v/v) and subjected to the lyophilization conditions as set out below.

### Lyophilization

Lyophilization was performed with a Christ alpha 2-4 LSC plus lyophilizer. The following lyophilization program according to Table 4 was applied.

**Table 4: Lyophilization program**

| | **temperature [°C]** | **pressure [mbar]** | **time [hh:mm]** |
|---|---|---|---|
| **freezing** | -30 | ambient | 12:00 |
| **main drying** | -30 | ambient - 0.120 (ramp) | 00:30 |
| | -30 | 0.120 | 10:00 |
| | -30 - -5 (ramp) | 0.120 | 02:00 |
| | -5 | 0.120 | 10:00 |
| **post drying** | 40 | 0.120 - 0.0010 (ramp) | 04:00 |
| | 40 | 0.0010 | 50:00 |

A representative powder X-ray diffractogram of amorphous baricitinib dicitrate prepared according to the above described process is displayed in Figure 4.

### Comparative Example 1

The solubilities of baricitinib free base, phosphate, trifluoroacetate, monocitrate and dicitrate in 0.1N HCl have been determined. For the compounds with known polymorphism one representative polymorph according to Table 5 has been used. A saturated solution of the compounds listed in Table 5 in 0.1N aqueous HCl solution has been prepared and the suspension was stirred at controlled temperature of 37 °C for 1 hour. Thereafter, the solid was removed by filtration and the baricitinib concentration of the mother liquor was determined by HPLC. PXRD of the isolated solid confirmed that no solid state changes occurred during the slurry.

| | |
|---|---|
| HPLC system | : e.g. Agilent 1100 |
| Column | : YMC-Pack Pro C18 RS, 3 microm, 4.6 × 150 mm |
| Eluent system | : Eluent A: 3.88g sulfamic acid, fill up with water to 1000 g |
| | : Eluent B: 3.88 g sulfamic acid, fill up with water to 300 g and add 587 g acetonitrile |
| Injection volume | : 2.5 microL |
| Flow rate | : 0.8 mL/min |
| Oven temperature | : 40 °C |
| Stop time | : 16 min (post-time 3 min) |
| Detection | : lambda = 205 nm |
| System | : gradient |

Gradient:

| **t (min)** | %A | %B |
|---|---|---|
| **0** | 90 | 10 |
| **10** | 0 | 100 |
| **15** | 0 | 100 |
| **16** | 90 | 10 |

The absolute solubility values were calculated from a calibration curve generated with solutions of known baricitinib concentrations and are listed in Table 5.

**Table 5: Comparative solubility data in 0.1N aqueous hydrochloric acid**

| **Baricitinib solid form used** | **Solubility in 0.1N aqueous HCl after 1 hour** |
|---|---|
| Crystalline free base of WO 2015/166434 A1 | 25 mg/mL |
| Phosphate salt of WO 2009/114512 A1 | 26 mg/mL |
| TFA salt Form A of CN105566332 | 29 mg/mL |
| Crystalline monocitrate of present invention | > 50 mg/mL |
| Crystalline dicitrate of present invention | > 50 mg/mL |

As can be seen from Table 5 the phosphate and trifluoroacetate salts of baricitinib show only a slightly increased solubility in 0.1N aqueous hydrochloric acid. In contrast, the solubilities of baricitinib mono- and dicitrate of the present invention are more than 2-fold higher than the solubility of the free base.

## Claims

1. Baricitinib citrate.

2. The baricitinib citrate of claim 1, which is a monocitrate **characterized by** the chemical structure according to Formula (II) 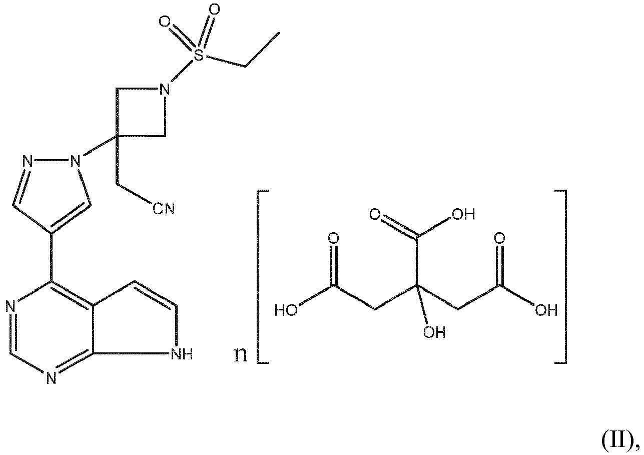 wherein n is in the range of from 0.7 to 1.3.

3. The baricitinib monocitrate according to claim 2 in crystalline or amorphous form.

4. The crystalline form of baricitinib monocitrate according to claim 3 **characterized by** having a powder X-ray diffractogram comprising reflections at 2-theta angles of (4.6 ± 0.2)°, (14.7 ± 0.2)°, (16.0 ± 0.2)°, (18.2 ± 0.2)° and (26.4 ± 0.2)°, when measured at a temperature in the range of from 20 to 30 °C with Cu-Kalpha_{1,2} radiation having a wavelength of 0.15419 nm.

5. The amorphous form of baricitinib monocitrate according to claim 3 **characterized by** a powder X-ray diffractogram comprising no reflections in the range of from 2 to 40° 2-Theta, when measured at a temperature in the range of from 20 to 30 °C with Cu-Kalpha_{1,2} radiation having a wavelength of 0.15419 nm.

6. The baricitinib citrate of claim 1, which is a dicitrate **characterized by** the chemical structure according to Formula (II) 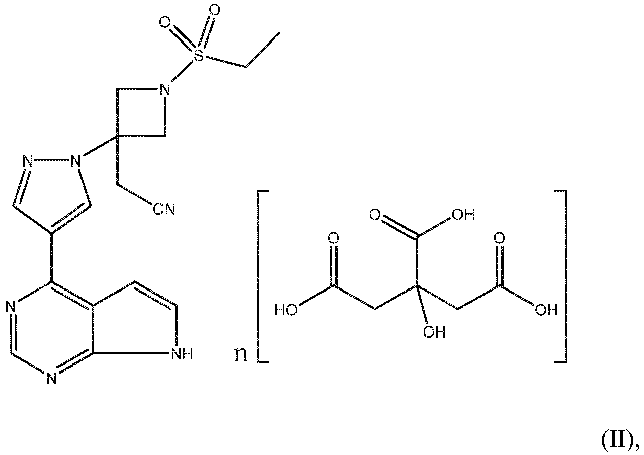 wherein n is in the range of from 1.7 to 2.3.

7. The baricitinib dicitrate of claim 6 in crystalline or amorphous form.

8. The crystalline baricitinib dicitrate of claim 7 **characterized by** having a powder X-ray diffractogram comprising reflections at 2-theta angles of (5.1 ± 0.2)°, (14.5 ± 0.2)°, (16.5 ± 0.2)°, (23.1 ± 0.2)° and (24.2 ± 0.2)°, when measured at a temperature in the range of from 20 to 30 °C with Cu-Kalpha_{1,2} radiation having a wavelength of 0.15419 nm.

9. The amorphous form of baricitinib dicitrate according to claim 7 **characterized by** a powder X-ray diffractogram comprising no reflections in the range of from 2 to 40° 2-Theta, when measured at a temperature in the range of from 20 to 30 °C with Cu-Kalpha_{1,2} radiation having a wavelength of 0.15419 nm.

10. A pharmaceutical composition comprising baricitinib citrate as defined in any one of claims 1 to 9 and one or more pharmaceutically acceptable excipient(s).

11. Baricitinib citrate as defined in any one of claims 1 to 9 or the pharmaceutical composition according to claim 10 for use as a medicament, in particular for use in the treatment of rheumatoid arthritis.

12. Process for the preparation of the crystalline baricitinib monocitrate as defined in claim 3 or 4 comprising:
(i) suspending amorphous baricitinib monocitrate in one or more aprotic solvent(s); or
(ii) reacting one mol equivalent baricitinib free base with 0.7 to 1.3 mol equivalent citric acid in one or more aprotic solvent(s); and
(iii)slurrying the mixture obtained in step (i) or (ii)

13. Process for the preparation of amorphous baricitinib monocitrate as defined in claim 3 or 5 comprising:
(i) reacting one mol equivalent baricitinib free base with 0.7 to 1.3 mol equivalent citric acid in the presence of one or more aqueous water miscible organic solvent(s) or 1,4-dioxane; and
(ii) lyophilizing the solution provided in step (i)

14. Process for the preparation of crystalline baricitinib dicitrate as defined in claim 7 or 8 comprising the steps of:
(i) suspending amorphous baricitinib dicitrate in one or more aprotic solvent(s); and
(ii) slurrying the mixture obtained in step (i)

15. Process for the preparation of amorphous baricitinib dicitrate as defined in claim 7 or 9 comprising:
(i) reacting one mol equivalent baricitinib free base with 1.8 to 6.0 mol equivalent citric acid in the presence of one or more aqueous water-miscible organic solvent(s) or 1,4-dioxane; and
(ii) lyophilizing the solution provided in step (i)
